Europäisches Patentamt

European Patent Office

Office européen des brevets

(19)

(11) **EP 0 967 483 A2**

(12) **EUROPÄISCHE PATENTANMELDUNG**

(43) Veröffentlichungstag:
29.12.1999 Patentblatt 1999/52

(51) Int. Cl.⁶: **G01N 33/22**, G01N 33/00

(21) Anmeldenummer: 98203278.1

(22) Anmeldetag: 29.09.1998

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **20.05.1998 EP 98201675**

(71) Anmelder:
• **N.V. NEDERLANDSE GASUNIE**
  **NL-9727 KC Groningen (NL)**
• **RUHRGAS AKTIENGESELLSCHAFT**
  **45138 Essen (DE)**

(72) Erfinder:
• **Jaeschke, Manfred Dr.**
  **46284 Dorsten (DE)**
• **Schouten, J.A. Prof. Dr.**
  **1018 XE Amsterdam (NL)**
• **van Wesenbeeck, P.J.J.M. Ir.**
  **9700 MA Groningen (NL)**

(74) Vertreter:
**Krijgsman, Willem et al**
**DSM Patents & Trademarks**
**Office Geleen**
**P.O. Box 9**
**6160 MA Geleen (NL)**

(54) **Verfahren zur verbrennungslosen Bestimmung des Brennwertes von Brenngas**

(57) Verfahren zur verbrennungslosen Messung des Brennwertes von Brenngas, wobei

a) die Schallgeschwindigkeit des Gases unter ersten und unter zweiten Referenzbedingungen bestimmt wird und eine der Meßgrößen Dielektrizitätskonstante, Schallgeschwindigkeit unter dritten Referenzbedingungen oder Kohlenstoffdioxidanteil des Brenngases erfaßt werden und
b) aus diesen drei Parametern der Brennwert abgeleitet wird wobei, vorzugsweise den Verfahrensschritten a) und b) mehrere Meßzyklen vorgeschaltet werden, bei denen der Schritt a) mit mehreren Referenzgasen bekannten Brennwerts durchgeführt wird; aus dem Verhältnis der bei den Referenzgasen erfaßten verschiedenen Meßsignale eine der Zahl der Meßzyklen entsprechende Zahl von Referenzsignalmustern in Zuordnung zu den bekannten Brennwerten gespeichert wird; und das Signalmuster aus einem späteren Meßzyklus an Brenngas unbekannten Brennwertes mit den Referenzsignalmustern zur Zuordnung eines bestimmten Brennwertes verglichen wird.

Fig. 3

EP 0 967 483 A2

**Beschreibung**

Die Erfindung bezieht sich auf ein Verfahren zur verbrennungslosen Messung des Brennwertes von Brenngas und auf eine Verwendung des Verfahrens zur verbrennungslosen Messung und/oder Regelung der Wärmemengenzufuhr zu Gasverbrauchseinrichtungen, insbesondere zu Erdgasverbrauchseinrichtungen. Die verbrennungslosen Verfahren zur Messung des Brennwertes bzw. zur Wärmemengemessung haben gegenüber kalorimetrischen Verfahren, in denen eine kontrollierte Teilstrom Verbrennung des übertragenen Gasstroms durchgeführt wird, den Vorteil, daß sie wesentlich kostengünstiger sind. Allerdings ist die technische Realisierung häufig aufwendig, abgesehen davon, daß Schwierigkeiten bei der Eichung auftreten können.

Zu den verbrennungslosen Meßverfahren gehören indirekte und korrelative Verfahren. Bei den indirekten Verfahren wird die Gaszusammensetzung analysiert. Aus der Zusammensetzung des Gases kann dann mit den Brennwerten für die reinen Stoffe der Brennwert des Brenngases bestimmt werden. Diese Verfahren (z.B. die Gaschromatographie) liefern sehr genaue Ergebnisse, sind aber technisch kompliziert und daher für den Einsatz in beispielsweise Haushalten kaum geeignet. Zudem sind sie störanfällig. Bei den korrelativen Verfahren zur Messung des Brennwertes bzw. zur Wärmemengemessung wird ein Zusammenhang zwischen einer leicht meßbaren physikalischen oder chemischen Meßgröße und dem Brennwert ausgenutzt. Die technische Durchführung ist hierbei einfacher, jedoch werden die Reproduzierbarkeit und die Genauigkeit der Messung des Brennwertes bzw. der Warmemenge in unerwünschtem Maße eingeschränkt.

Aufgabe der Erfindung ist es, den Aufwand bei der korrelativen verbrennungslosen Messung des Brennwertes bzw. der Messung und/oder Regelung der Wärmemengenzufuhr zu Verbrauchseinrichtungen weiter zu verringern und insbesondere ein zuverlässiges und genaues Meßverfahren zur Verfügung zu stellen. Gelöst wird diese Aufgabe erfindungsgemäß dadurch, daß bei dem eingangs genannten Verfahren

   a) die Schallgeschwindigkeit des Gases unter ersten und unter zweiten Referenzbedingungen bestimmt wird und eine der Meßgrößen Dielektrizitätskonstante, Schallgeschwindigkeit unter dritten Referenzbedingungen oder Kohlenstoffdioxidanteil des Brenngases erfaßt werden und
   b) aus diesen drei Parametern der Brennwert abgeleitet wird.

Besonders genaue Ergebnisse lassen sich für Brenngase erzielen, deren Brennwert bei Normbedingungen zwischen 20 und 48 Mj/m3 liegt, deren auf trockene Luft bezogene relative Dichte zwischen 0,55 und 0,9 betragt, deren Kohlenstoffdioxidanteil kleiner gleich 0,3 ist und deren Wasserstoff- und Kohlenmonoxidanteil kleiner als 0,1 bzw. 0,03 ist. Als Meßbedingungen sind Temperaturen im Bereich von 225 bis 350 K und Drücke kleiner gleich 60 MPa besonders geeignet.

Als erste Referenzbedingungen werden vorzugsweise Normbedingungen eingestellt (273,15 K und 1 bar) oder ein Druck zwischen 1 und 10 bar, mehr bevorzugt zwischen 3 und 7 bar. Obwohl die Temperatur nicht sehr kritisch ist und innerhalb eines weiten Bereiches gewählt werden kann, liegt aus technischen Gründen die Temperatur oberhalb 225 K, beispielswiese zwischen 270 K und 295 K. Unter zweiten Referenzbedingungen wird bevorzugt ein Druck über 30 bar eingestellt. Obwohl die Temperatur nicht sehr kritisch ist und innerhalb eines weiten Bereiches gewählt werden kann, liegt auf technischen Gründen die Temperatur zwischen 225 K und 350 K. Meist bevorzugt sind die Betriebsbedingungen für diesen Parameter. Die bevorzugten Werte für die dritten Referenzbedingungen sind ein Druck über 150 bar, mehr bevorzugt über 175 bar, beispielsweise 200 bar. Bei der Verwendung einer dritten Referenzbedingung soll der Druck der zweiten Referenzbedingung bevorzugt unterhalb 70 bar eingestellt werden.

Die Schallgeschwindigkeit bei den genannten Referenz-, einschließlich Betriebsbedingungen kann in einer separaten Meßeinheit, beispielsweise über die Resonanzfrequenz von Wirbelpfeifen oder von Hohlkörpern oder eine Weg-Zeit-Messung z.B. in Ultraschallzählern erfaßt werden. Die Dielektrizitätskonstante läßt sich auch bei Betriebsbedingungen kostengünstig und mit hoher Genauigkeit erfassen. Das Kohlenstoffdioxidanteil ist unter allen erwähnten Bedingungen einfach zu bestimmen mit bekannten Meßgeräten, z.B. über die Messung der Lichtabsorption im Infrarotbereich.

Folglich können die jeweils erforderlichen drei Messungen ohne großen technischen Aufwand, zuverläßssig und genau durchgeführt werden, so daß die Verknüpfung der Meßwerte entsprechende Ergebnisse für den Brennwert liefert. Der so ermittelte Brennwert kann z.B. zur Steuerung von Verbrennungsprozessen verwendet werden.

Je nach Anwendungsfall kann der volumenbezogene Brennwert unter Referenz- oder Betriebsbedingungen, der spezifische (massenbezogene) Brennwert bzw. der molare Brennwert bestimmt werden. Insgesamt gibt es drei Variationen des erfindungsgemäßen Verfahrens zur Messung des Brennwertes von Brenngas.

Bei alle drei Varianten wird die Schallgeschwindigkeit unter ersten und unter zweiten Referenzbedingungen erfaßt.

Bei der ersten Variante wird zusätzlich noch die Schallgeschwindigkeit unter dritten Referenzbedingungen erfaßt. Das Erfassen von zwei und in dieser ersten Variante sogar drei Schallgeschwindigkeiten hat das Vorteil, dass alle Messungen in einer Meßeinrichtung durchgeführt werden können. Der Druck in dem Gerät kann variiert werden durch Zusammendrücken oder Ausdehnenlassen des Messvolumens. Beim Zusammendrücken oder Ausdehnen ändert sich auch die Temperatur des Brenngases, womit das Einstellen veränderter Referenzbedingungen erleichtert werden kann. Wenn erwünscht, können der Meßeinrichtung zur Bestimmung der Schallgeschwindigkeiten noch Mittel zugeordnet sein zur veränderten Einstellung der Temperatur. Bei der zweiten Variante wird zusätzlich zu den zwei Schallgeschwindigkeiten noch die Dielektrizitätstatskonstante, zum Erreichen einer großen Genauigkeit vorzugsweise bei einem Druck von wenigstens 10 bar, z.B. bei zweiten Referenzbedingungen, z.B Betriebsbedingungen, gemessen.

Bei der dritten Variante wird zusätzlich zu den zwei Schallgeschwindigkeiten noch der Kohlenstoffdioxidanteil des Brenngases bestimmt. Die Bestimmung der Dielektrizitätskonstante und des Kohlenstoffdioxidanteils kann durchgeführt werden in derselbe Meßumgebung wie diejenige in der die Schallgeschwindigkeiten bestimmt werden. Dieses ermöglicht eine sehr kompakte Meßeinrichtung.

Vorteilhafterweise werden die Schallgeschwindigkeit unter zweiter Referenzbedingungen und die Dielektrizitätskonstante oder der Kohlenstoffdioxidanteil unter gleichen Referenzbedingungen, vorzugsweise unter Betriebsbedingungen, in einer gemeinsamen Meßumgebung erfaßt. Auf diese Weise wird nur eine Temperatur- und Druckmessung und folglich nur ein Thermostat zur Herstellung bzw. Einhaltung der Referenzbedingungen benötigt. Außerdem erhöhen einheitliche Referenzbedingungen für die unterschiedenen Messungen die Genauigkeit, mit der die Wärmemengenzufuhr bestimmt werden kann.

Das Bestimmen von wenigstens zwei Schallgeschwindigkeiten bietet den weitere Vorteil, daß auf die Bestimmung der Dichte des Brenngases verzichtet werden kann. Dichtemessungen sind, insbesondere unter Betriebsbedingungen, kostspielig und aufwendig. Beim Verfahren laut der Erfindung wird vorzugsweise, insbesondere wenn als dritte Meßgröße das Kohlenstoffdioxidanteil erfaßt wird, keine zusätzliche Dichtemessung durchgeführt.

Zur Festlegung der Referenzbedingungen werden die Meßgrößen Temperatur und Druck benötigt. Diese können im Schritt a) zusätzlich erfaßt werden. Wenn eine geringeren Meßgenauigkeit zulässig ist, können für diesen Parameter auch aus der Praxis geschätzte Werte benutzt werden. Die Meßgenauigkeit kann dadurch weiter erhöht werden, daß im Schritt a) zusätzlich der Stickstoffanteil erfaßt wird.

Zum Auffinden einer geeigneten Korrelation zwischen den gemessenen Parametern und dem Brennwert ist es vorteilhaft, den jeweiligen Verfahrensschritten a) und b) wenigstens einmal mehrere Meßzyklen vorzuschalten, bei denen der Verfahrensschritt a) mit mehreren Referenzgasen bekannten Brennwerts durchgeführt wird. An dem Referenzgas werden dann die für die verschiedenen Varianten der Verfahren benötigten Meßgrößen erfaßt. In diesen Referenzzyklen wird aus dem Verhältnis der erfaßten verschiedenen Meßsignale eine der Zahl der Meßzyklen entsprechende Zahl von Referenzsignalmustern in Zuordnung zu den bekannten Brennwerten gespeichert. Das Signalmuster aus einem späteren Meßzyklus an Brenngas unbekannten Brennwertes wird mit den Referenzsignalmustern zur Zuordnung eines bestimmten Brennwertes verglichen.

Zur Erhöhung der Referenzgenauigkeit sollte eine Vielzahl von Referenzzyklen durchgeführt werden, in denen nacheinander die verschiedenen Meßgrößen über den zu erwartenden Meßbereich variiert werden. Eine eindeutige und genaue Zuordnung eines bestimmten Brennwertes zu einem in einem Meßzyklus erfaßten Signalmuster eines Brenngases wird durch Interpolation der verschiedenen Referenzsignalmuster erzielt.

Ein wesentlicher Vorteil besteht darin, daß die Korrelation zwischen Brennwert und gemessenen Parametern mit Hilfe einer beliebigen Anzahl von Referenzzyklen für eine spezielle Anwendung nur einmal aufgefunden werden muß. Der einmalige Aufwand ist verhältnismäßig gering. Die Referenzbedingungen sollten hierbei möglichst getreu den später zu erwartenden Meßbedingungen gewählt werden. Es sollten also für alle Meßgrößen nur die tatsächlich in Frage kommenden Meßbereiche mit einer ausreichenden Genauigkeit als Referenzsignalmuster erfaßt werden.

Je nachdem die Zusammensetzung des Brenngases größere Variationen aufweisen mag, müssen im allgemeinen mehr Referenzsignalmuster bestimmt werden. Ein zusätzliches Vorteil der Wahl von Schallgeschwindigkeiten als die zubestimmenden Meßgrössen in dem erfundenen Verfahren ist, daß bezüglich der Abhängigkeit der Gaszusammensetzung von der Schallgeschwindigkeit bereits sehr viele Daten zur Verfügung stehen und das auch weiterhin diese Daten experimentel gut zu bestimmen sind. Benutzung dieser bereits zur Verfügung stehenden Daten ermöglicht also die Berechnung des Brennwertes und Schallgeschwindigkeiten in Abhängigkeit von der Gaszusammensetzung im relevanten Bereich. Also können diese Daten zur rechnerischen Bestimmung des Brennwertes aus den gemessenen Parametern verwendet werden.

Eine bevorzugte Weiterbildung der Erfindung ist dadurch gekennzeichnet, daß im Rahmen der rechnerischen Bestimmung des Brennwertes der jeweilige Anteil einer vorgegebenen Anzahl von Alkanen, einschließlich Methan, dadurch bestimmt wird, daß der Anteil der einzelnen Alkane, ausgenommen Methan, mit Hilfe jeweils einer zugehörigen, von einer ausgewählten physikalischen Meßgröße, vorzugsweise dem molaren Brennwert, der Summe der vorgegebenen Alkane abhängigen Funktion bestimmt wird und daß der Methananteil aus der Differenz zwischen dem Anteil der Summe der vorgegebener Alkane und der Summe der durch die Funktionen bestimmter Anteile der Alkane bestimmt wird.

Als vorgegebene Alkane sollten möglichst alle im Brenngas tatsächlich vorhandenen Alkane gewählt und vorgegeben werden.

Es hat sich gezeigt, daß die Anteile der Alkane bei natürlichen Brenngasen stets in einem bestimmten Verhältnis zueinander stehen, welches lediglich von einer physikalischen Megröße, z. B. dem molaren Brennwert, der Summe der vorgegebenen Alkane abhängt. Dies ist offenbar darauf zurückzuführen, daß Erdgase in ihrer natürlichen Form stets eine Gleichgewichtsphase durchlaufen haben, in welcher ihr Gas- oder Flüssigkeitsphasen im Gleichgewicht zueinander standen. Jedoch ist das Verfahren nicht auf die natürlichen Brenngase, und zwar mit oder ohne Kokereigaszusatz, beschränkt. Für synthetische Gase mit Zusätzen oder für Gasgemische mit vielen Komponenten ist die Unsicherheit bei der Bestimmung der Gaszusammensetzung lediglich etwas größer.

Der molare Brennwert der Summe der vorgegebenen Alkane kann beispielsweise wiederum mit Hilfe von Referenzsignalzyklen bestimmt werden. Da bei den Referenzgasen die Zusammensetzung bekannt ist, ist auch ihr molarer Brennwert der Summe der vorgegebenen Alkane bekannt. Folglich kann aus dem Verhältnis der bei den Referenzgasen erfaßten Meßsignale eine der Zahl der Referenzmeßzyklen entsprechende Zahl von Referenzsignalmustern in Zuordnung zu den bekannten molaren Brennwerten der Summe der vorgegebenen Alkane gespeichert werden. Bei einem späteren Meßzyklus kann der molare Brennwert der Summe der vorgegebenen Alkane des Brenngases lediglich durch Vergleich der erfaßten Meßsignale mit den gespeicherten Referenzsignalmustern bestimmt werden.

Vorteilhafterweise können als Funktionen zur Bestimmung der Anteile der einzelnen Alkane, ausgenommen Methan, Polynome, vorzugsweise 2. Ordnung, verwendet werden.

Bei einem bevorzugten Ausführungsbeispiel wird der Methan-, Ethan-, Propan-, Isobutan-, n-Butan-, Isopentan-, n-Pentan-, Hexan-, Heptan- und Oktananteil mit Hilfe der Funktionen bestimmt. Es hat sich gezeigt, da der Anteil aller weiteren Kohlenwasserstoffe, insbesondere bei natürlichem Brenngas, vernachlässigt werden kann. Der Zusammenhang zu dem molaren Brennwert der Summe der vorgegebenen Alkane lautet in diesem Fall z.B.:

$$X_{C2H6} = [\alpha_1 (H_{CH} - H_{CH4}) + \beta_1 (H_{CH} - H_{CH4})^2] X_{CH} \qquad (1.1)$$

$$X_{C3H8} = [\alpha_2 (H_{CH} - H_{CH4}) + \beta_2 (H_{CH} - H_{CH4})^2] X_{CH} \qquad (1.2)$$

$$X_{i-C4H10} = [\alpha_3 (H_{CH} - H_{CH4}) + \beta_3 (H_{CH} - H_{CH4})^2] X_{CH} \qquad (1.3)$$

$$X_{n-C4H10} = [\alpha_4 (H_{CH} - H_{CH4}) + \beta_4 (H_{CH} - H_{CH4})^2] X_{CH} \qquad (1.4)$$

$$X_{i-C5H12} = [\alpha_5 (H_{CH} - H_{CH4}) + \beta_5 (H_{CH} - H_{CH4})^2] X_{CH} \qquad (1.5)$$

$$X_{n-C5H12} = [\alpha_6 (H_{CH} - H_{CH4}) + \beta_6 (H_{CH} - H_{CH4})^2] X_{CH} \qquad (1.6)$$

$$X_{n-C6H14} = [\alpha_7 (H_{CH} - H_{CH4}) + \beta_7 (H_{CH} - H_{CH4})^2] X_{CH} \qquad (1.7)$$

$$X_{n-C7H16} = [\alpha_8 (H_{CH} - H_{CH4}) + \beta_8 (H_{CH} - H_{CH4})^2] X_{CH} \qquad (1.8)$$

$$X_{n-C8H18} = [\alpha_9 (H_{CH} - H_{CH4}) + \beta_9 (H_{CH} - H_{CH4})^2] X_{CH} \qquad (1.9)$$

Hierbei sind $\alpha_i$ und $\beta_i$ Konstanten und $H_{CH4}$ der molare Brennwert von Methan. Die Variable $H_{CH}$ steht für den molaren Brennwert der Summe der vorgegebenen Alkane ($H_{CH} = \Sigma X_{CH,i} H_{CH,i}$). Der Methananteil wird in diesem Fall wie folgt bestimmt:

$$X_{CH4} = X_{CH} - (X_{C2H6} + X_{C3H8} + X_{i-C4H10} + X_{n-C4H10} + X_{i-C5H12} + X_{n-C5H12} + X_{n-C6H14} + X_{n-C7H16} + X_{n-C8H18}) \qquad (2)$$

Eine Weiterbildung der Erfindung ist dadurch gekennzeichnet, daß den Verfahrensschritten a) und b) mehrere Meßzyklen vorgeschaltet werden, bei denen der Schritt a) mit mehreren Referenzgasen durchgeführt wird, deren Zusammensetzung und deren ausgewählte physikalische Meßgröße der Summe der vorgegebenen Alkane bekannt ist, daß aus den bei den Referenzgasen erfaßten Meßsignalen die Konstanten, z.B. Koeffizienten, der den Anteil der Alkane, ausgenommen Methan, beschreibenden Funktionen bestimmt werden, daß die Konstanten der Funktionen in Zuordnung zu den jeweiligen Alkanen gespeichert werden und daß der Anteil der Alkane, ausgenommen Methan, aus einem späteren Meßzyklus an Brenngas unbekannter Zusammensetzung mit Hilfe der Funktionen bestimmt wird.

Auf diese Weise können die Konstanten $\alpha_i$ und $\beta_i$ mit Hilfe bereits von zwei Referenzzyklen für alle natürlichen Erdgase aufgefunden werden. Zur Erhöhung der Meßgenauigkeit, können beliebig viele Referenzzyklen durchgeführt werden. Der Aufwand bleibt auch bei einer großen Anzahl von Referenzzyklen verhaltnismäßig gering, da die Konstanten $\alpha_i$ und $\beta_i$ nur einmal bestimmt werden müssen.

Der Anteil der Summe der Alkane des Brenngases kann unter der Annahme bestimmt werden, daß das Brenngas nur aus einer vorgegebenen Anzahl von Alkanen, aus Stickstoff und aus Kohlenstoffdioxid besteht. Die zugehörigen Gleichungen haben folglich die Form:

$$X_{CH} = 1 - X_{N2} - X_{CO2} \qquad (3)$$

Hierbei geben $X_{N2}$ und $X_{CO2}$ den Anteil von Stickstoff bzw. Kohlenstoffdioxid an.

Zur Erhöhung der Meßgenauigkeit kann alternativ angenommen werden, daß das Brenngas außerdem Wasserstoff und/oder Kohlenstoffmonoxid enthält. Werden sowohl der Wasserstoffals auch der Kohlenstoffmonoxidanteil berücksichtigt, so lautet die Gleichung (3):

$$X_{CH} = 1 - X_{N2} - X_{CO2} - X_{H2} - X_{CO} \qquad (3')$$

Ein bevorzugtes Ausführungsbeispiel ist dadurch gekennzeichnet, daß die Schallgeschwindigkeiten unter drei unterschiedliche Bedingungen aus der Gaszusammensetzung abgeleitet werden. Diese Schallgeschwindigkeiten lassen sich auf einfache Weise aus der Zusammensetzung des Gases errechnen mit Hilfe von einer Zustandsgleichung wie z.B. die im Gasfach oft verwendete AGA-8 Gleichung.

$$W_{i,ber} = f(p_i, T_i, X_{CH4}, ... X_{n-C8H18}, X_{CO2}, X_{N2}) \qquad (4)$$

Ein Beispiel einer Weiterbildung der Erfindung ist dadurch gekennzeichnet, daß aus der Zusammensetzung des Brenngases Werte für Schallgeschwindigkeiten unter drei unterschiedlichen Meßbedingungen abgeleitet werden, daß die Differenz zwischen dem abgeleiteten und dem erfaßten Wert der Schallgeschwindigkeiten gebildet wird, daß falls die Differenz einen vorgegebenen Schwellwert überschreitet, der Anteil wenigstens einer der zu bestimmenden Komponenten des Brenngases abgeändert festgesetzt wird, daß auf der Basis des abgeänderten Wertes oder der abgeänderten Werte erneut die Zusammensetzung errechnet wird, die Werte der ausgewählten Parameter erneut errechnet werden und die Differenz zur Meßwerten bestimmt wird und daß die letzten beiden Schritte solange wiederholt werden, bis die Differenz unterhalb des Schwellwertes liegt.

Es hat sich gezeigt, daß auf diese Weise die Gaszusammensetzung besonders schnell bestimmt werden kann. Sollen alle oben angegebenen 14 Gleichungen (1.1 bis 4) berücksichtigt werden, so enthalten diese bis zu 14 Unbekannte, nämlich $X_{CH}$, $X_{CH4}$, $X_{C2H6}$, $X_{C3H8}$, ... , $X_{n-C8H18}$, $W_{1,ber}$, $W_{2,ber}$, $W_{3,ber}$. Zur Lösung dieser 14 Gleichungen können ein oder mehrere Werte, Z.B. die drei erwähnte Schallgeschwindigkeiten abgeleitet und mit dem zugehörigen erfaßten Werte verglichen werden.

Vorteil des erfindungsgemäßen Verfahrens ist, daß nicht nur der Brennwert bestimmt werden kann, sondern das auch andere wichtigen Parameter des Gases wie Z.B. der Realgasfaktor, die Dichte, die Schallgeschwindigkeit, die Enthalpie, die Methanzahl oder der Wobbe-Indiz des Brenngases sich aus der Zusammensetzung errechnen lassen.

Die mit dem erfindungsgemäßen Verfahren bestimmten physikalischen Größen haben im wesentlichen die gleiche Güte wie die mit Hilfe einer Gaschromatographie bestimmten Werte. Erfindungsgemäß gelingt es mit Hilfe von lediglich drei Meßgrößen, z.B. zwei Schallgeschwindigkeiten und die Dielectrizitätskonstante, eine Vielzahl von verfahrenstechnischen Berechnungen durchzuführen. Zunächst können Zustandsänderungen in Gasspeichern bzw. Speichervolumen festgestellt werden. Außerdem können die relevanten Gastransportdaten, wie z.B. Temperatur oder Druckabfall ermittelt werden. Für erdgasbetriebene Fahrzeuge kann die erforderliche Auslegung der Gasbefüllstationen berechnet werden. Füllstandsmessungen können mit dem erfindungsgemäßen Verfahren kontrolliert und ausgelegt werden.

Auch im Zusammenhang mit Wärmetauschern ist die Erfindung von großem Vorteil. Die Auslegung von Wärmetauschern kann mit dem erfindungsgemäßen Verfahren berechnet werden.

Leistungsmessungen an Wärmetauschern können mit dem Verfahren ausgewertet werden. Schließlich können Verdichterkennfelder und Verdichterleistungen mit dem erfindungsgemäßen Verfahren bestimmt werden.

Bei sämtlichen vorgenannten Anwendungen sind bisher kostspielige Gaschromatographie-Untersuchungen erforderlich.

Auch die Methanzahl kann mit dem erfindungsgemäßen Verfahren bestimmt werden. Werden als Eingangsmeßsignale für das erfindungsgemäße Verfahren diejenigen Gasbeschaffenheitsdaten verwendet, die ursprünglich für Abrechnungszwecke gemessen wurden, so kann die Methanzahl im wesentlichen mit der gleichen Genauigkeit wie bei Einsatz eines Gaschromatographen bestimmt werden. Die Abweichung der Methanzahlen betragt weniger als 2%.

Bei Verwendung der für Abrechnungszwecke gemessenen Gasbeschaffenheitsdaten oder der Netzsimulationen gelingt es, ohne zusätzliche Messungen Brenngasabnehmer jederzeit über aktuelle und ggf. zukünftige Schwankungen der Methan-Zahl zu informieren. Das Gastransportnetz kann außerdem ohne Mehraufwand flexibler gesteuert werden.

Weitere vorteilhafte Ausführungsformen der Erfindung sind in den Unteransprüchen gekennzeichnet.

Im folgenden wird die Verwendung des erfindungsgemäßen Verfahrens zur verbrennungslosen Messung und/oder Regelung der Wärmengenzufuhr zu Gasverbrauchseinrichtungen anhand von in der Zeichnung dargestellten Ausführungsbeispielen näher erläutert. In der Zeichnung zeigen:

Fig. 1 ein Meßdiagramm, in dem der Stoffmengenanteil von Ethan und Propan für verschiedene Gase gegen den molaren Brennwert der Summe der Kohlenwasserstoffe aufgetragen ist, und

Fig. 2A und Fig. 2B ein Ablaufdiagramm zur Bestimmung der Gaszusammensetzung gemäß einem Ausführungsbeispiel des erfindungsgemäßen Verfahrens.

Fig. 3 eine schematische Ansicht einer Anordnung zur Durchführung eines ersten Ausführungsbeispiels der erfindungsgemäßen Verwendung des Verfahrens zur Brennwertmessung;

Fig. 4 eine schematische Ansicht einer Anordnung zur Durchführung eines zweiten Ausführungsbeispiels der erfindungsgemäßen Verwendung;

Fig. 5 eine schematische Ansicht einer Anordnung zur Durchführung eines dritten Ausführungsbeispiels der erfindungsgemäßen Verwendung;

Fig. 6 eine schematische Ansicht einer Anordnung zur Durchführung eines vierten Ausführungsbeispiels der erfindungsgemäßen Verwendung;

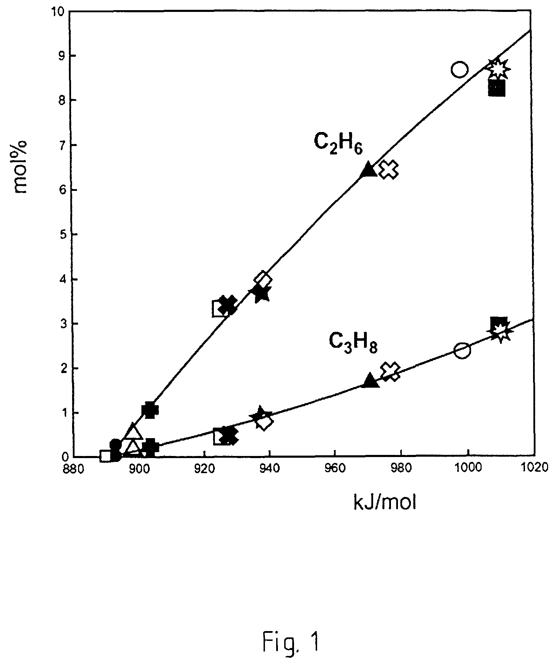

In Fig. 1 ist der molare Brennwert der Summe der Alkane($H_{CH}$) auf der x-Achse und der Stoffmengenanteil von Ethan ($C_2H_6$) und Propan ($C_3H_8$) auf der y-Achse aufgetragen. Für verschiedene natürliche Erdgase wurden die entsprechenden Meßwerte ermittelt und aufgetragen. Der Anteil von sowohl Ethan als auch Propan wurde durch ein Polynom 2. Ordnung angenähert. Wie die Fig. 1 zeigt, kann sowohl der Anteil von Ethan als auch von Propan überraschend gut durch ein vom molaren Brennwert der Summe der Alkane abhängiges Polynom 2. Ordnung angenähert werden. Entsprechendes gilt für die weiteren Alkane bis zum Octan. Die Meßwerte für diese Alkane sind aus Gründen der Obersichtlichkeit in Fig. 1 nicht dargestellt.

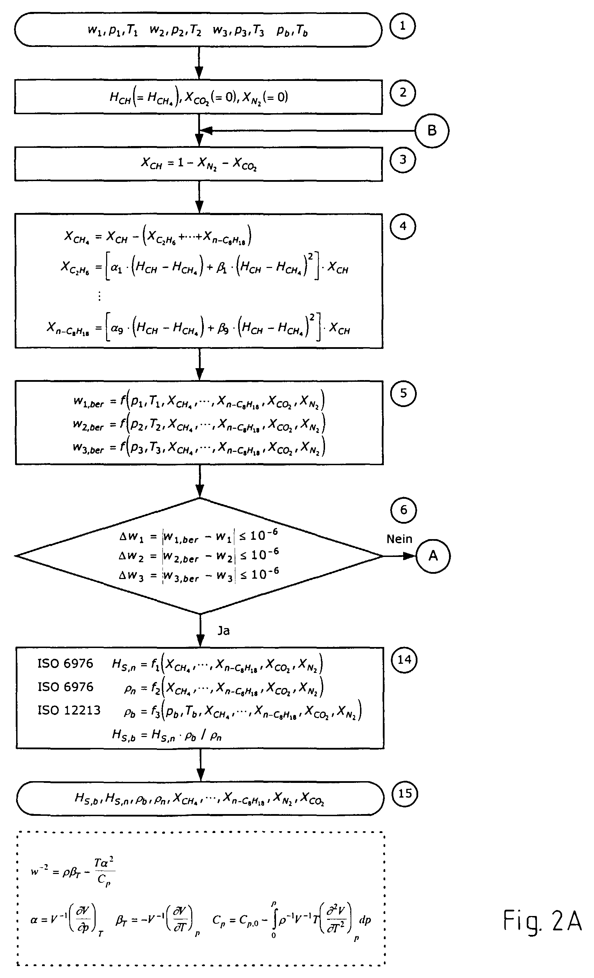

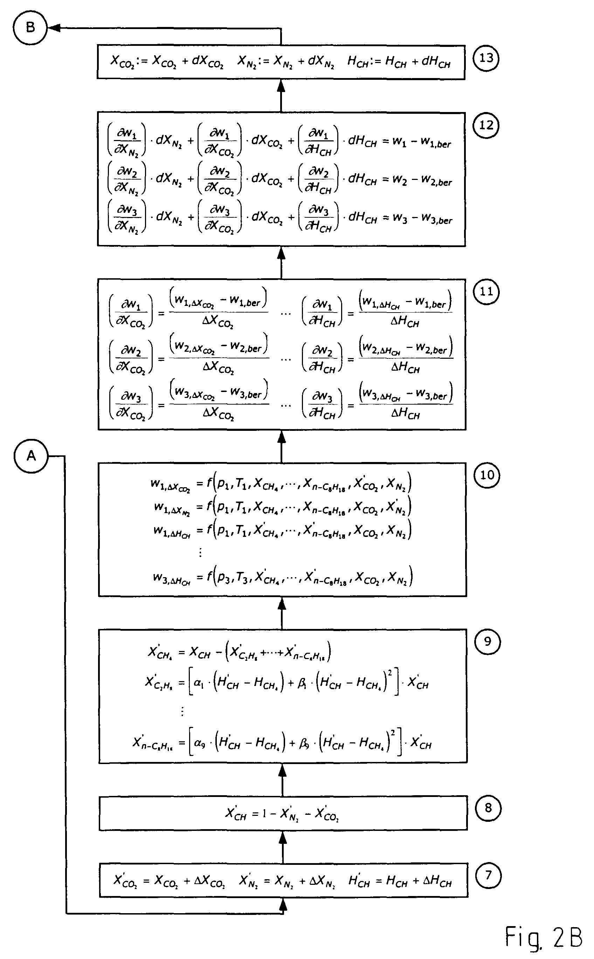

Fig. 2A und 2B zeigen ein Ablaufdiagramm zur Bestimmung der Gaszusammensetzung gemäß einem bevorzugten Ausführungsbeispiel.

Im Schritt 1 werden drei Schallgeschwindigkeiten, je unter unterschiedlichen Bedingungen, gemessen. Von den Bedingungen wird der Druck jeweils eingestellt, die Temperatur kann eingestellt werden oder die Messung kann stattfinden bei der Temperatur die sich nach einer eventuellen Druckänderung eingestellt hat. Den tatsächliche Druck und die Temperatur von jeder Messung werden gemessen.

Im Schritt 2 werden Startwerte für den molaren Brennwert der Summe der Alkane ($H_{CH}$), den Stickstoffanteil $X_{N2}$, und den Kohlenstoffdioxidanteil $X_{CO2}$ festgelegt. Für die Startwerte kann man auch die Daten reines Methanes benutzen, d. h. $X_{CO2} = 0$, $X_{N2} = 0$ und $H_{CH} = H_{CH4}$. In der Praxis werden mit diesen Startwerten gute Ergebnisse erzielt.

Aus den Startwerten für den Stickstoffanteil und den Kohlendioxidanteil wird dann im Schritt 3 der Anteil der Summe der Alkane $X_{CH}$ bestimmt.

Im Schritt 4 wird dann mit Hilfe des molaren Brennwerts der Summe der Alkane $H_{CH}$ der Anteil der Alkane, ausgenommen Methan, mit Hilfe der Gleichungen (1.1) bis (1.9) bestimmt. Folglich wird der Methananteil $X_{CH4}$ gemäß Gleichung (2) bestimmt. Mit Hilfe einer Zustandsgleichung werden im Schritt 5 aus der berechneten Zusammensetzung des Gases Werte der Schallgeschwindigkeit unter den gleichen Bedingungen wie die im Schritt 1 erfaßten Meßwerte berechnet. Dies kann zum Beispiel geschehen mit Hilfe der bekannten AGA8-DC92 Zustandsgleichung.

Im Schritt 6 wird festgestellt, ob der Betrag der Differenz zwischen den berechneten Werten der Schallgeschwindigkeit $w_{1,ber}$, $w_{2,ber}$, $w_{3,ber}$ und den im Schritt 1 erfaßten Meßwerten $w_1$, $w_2$, $w_3$ für jeden Wert kleiner als der auf $10^{-6}$ festgelegte Schwellwert ist. Falls nein, wird das Verfahren mit dem Schritt 6 fortgesetzt.

In den Schritten 7 bis 11 wird die Empfindlichkeit bzw. Sensitivity der Eingangsparameter $X_{N2}$, $X_{CO2}$, und $H_{CH}$ bestimmt. Im Schritt 7 werden dazu zuerst für $\Delta X_{N2}$, $\Delta X_{CO2}$, und $\Delta H_{CH}$ Werte festgesetzt. Gute Ergebnisse werden erzielt mit 0,1 mol-% für $\Delta X_{N2}$ und $\Delta X_{CO2}$, und 1,0 MJ/mol für $\Delta H_{CH}$.

Im Schritt 8 wird aus den neuen Werten des Kohlenstoffdioxidanteils $X'_{CO2}$ und des Stickstoffanteils $X'_{N2}$ die Summe der Alkane $X'_{CH}$ nach Gleichung (3) berechnet.

Folglich wird im Schritt 9 mit Hilfe der neuen Summe der Alkane $X'_{CH}$ und des neuen molaren Brennwerts der Summe der Alkane $H'_{CH}$ der Anteil der Alkane, ausgenommen Methan, mit Hilfe der Gleichungen (1.1) bis (1.9) bestimmt und letztendlich wird der Methananteil $X'_{CH4}$ gemäß Gleichung (2) berechnet.

Im Schritt 10 werden die Werte der Schallgeschwindigkeit bestimmt unter den drei in Schritt 1 gegebenen unterschiedlichen Bedingungen. Dabei wird jedesmal nur eine der drei Eingangsparameter variert. Nach Vollendung des Schrittes 10 gibt es also für jede Bedingung Werte der Schallgeschwindigkeit in Abhängigkeit von $X'_{CO2}$, $X'_{N2}$ und $H'_{CH}$.

Darauf werden in Schritt 11 die partiellen Ableitungen bestimmt. Dazu wird zuerst die Änderung der Schallgeschwindigkeit in Abhängigkeit von einer der Eingangsparameter bestimmt. Dazu wird die Differenz bestimmt zwischen den in Schritt 10 berechneten Werten der Schallgeschwindigkeit, wobei einer der Eingangsparameter ein wenig abgeändert ist, und dem in Schritt 5 berechneten Wert. Die partielle Ableitung läßt sich jetzt einfach berechnen aus dem Quotient der obengenannten Änderung der Schallgeschwindigkeit und der in Schritt 6 festgelegte Änderung der betroffenen Eingangsparameter.

Im Schritt 12 werden allgemeingültige Gleichungen zur Beschreibung der Fortpflanzung von Änderungen der Eingangsgrößen in einer Ausgangsgröße angegeben. Nach Einsetzen der in Schritt 10 berechneten Werte der partiellen Ableitungen sowie der in Schritt 6 berechneten Abweichung der Ausgangsgrößen, kann das lineare Gleichungssystem mittels allgemein bekannter mathematischer Techniken nach den Größen $x_{N2}$, $x_{CO2}$ und $H_{CH}$ gelöst werden. Das Ergebnis sind Schätzungen der erforderlichen Änderungen der Parameter $X_{N2}$, $X_{CO2}$ und $H_{CH}$.

Im Schritt 13 werden dann die neue Werte für den Stickstoffanteil $X_{N2}$, den Kohlenstoffdioxidanteil $X_{CO2}$, und den molaren Brennwert der Summe der vorgegebenen Alkane HCH neu festgesetzt, und zwar indem die in Schritt 12 berechneten Abweichungen dieser drei Parameter, einfach zu den vorhergehende Wert addiert werden.

Mit dem neu festgesetzten Wert für den Stickstoffanteil werden die Schritte 3 bis 6 dann wiederholt. Falls im Schritt 6 erneut die Grenzwerte von $\Delta w_1$, $\Delta w_2$ und/oder $\Delta w_3$ überschritten wird, werden erneut die Schritte 7 bis 13 und 3 bis 6 durchgeführt. Erst wenn im Schritt 6 die Grenzwerte von $10^{-6}$ von $\Delta w_1$, $\Delta w_2$ und $\Delta w_3$ eingehalten werden, stehen die Anteile der Alkane sowie der Stickstoffanteil mit der gewunschten Genauigkeit fest.

In diesem letzten Fall werden dann in den Schritten 14 und 15 alle gewünschten Gasparameter berechnet. Dies geschieht mit Hilfe der bekannten ISO 6976 und ISO 12213.

Das beschriebene Verfahren kann analog durchgeführt werden für die Kombinationen von zwei Schallgeschwindigkeiten mit der Dielektrizitätskonstante bzw. dem Kohlenstoffdioxidanteil des Brenngases.

Die erfindungsgemäße Aufgabe wird ferner gelöst durch die Verwendung des erfindungsgemäßen Verfahrens zur verbrennungslosen Messung und/oder Regelung der Wärmemengenzufuhr zu Gasverbrauchseinrichtungen, insbesondere zu Erdgasverbrauchseinrichtungen, wobei im Schritt a) zusätzlich das Brenngas. bzw. ein Teilstrom des Brenngases durch einen Volumen- oder Massenzähler geleitet und das Volumen bzw. die Masse des zugeführten Brenngases gemessen wird.

Unter Gasverbrauchseinrichtungen werden alle benötigte Einrichtungen zur Verwendung von Gas bei privaten und industriellen Abnehmer sowie alle Übergabestellen o. dgl. verstanden.

Erfindungsgemäß kann z.B. die Wärmemengenzufuhr zu Haushalten bereits aus vier Parametern, nämlich erstens dem Volumen oder der Masse, zweitens zwei Schallgeschwindigkeiten und drittens der Dielektrizitätskonstante oder eine dritte Schallgeschwindigkeit oder das Kohlenstoffdioxidanteil unter den hieroben erwähnten Bedingungen abgeleitet werden. Der technische Aufwand und die Kosten hierfür sind minimal.

Zur Erhöhung der Genauigkeit können, wie bei der Messung des Brennwertes, beliebig viele weitere Meßgrößen erfaßt werden. Für Anwendungsfälle in denen besonders hohe Meßgenauigkeiten erforderlich sind, z.B. für die Bestimmung der Wärmemengenzufuhr an Übergabestellen von Haupttransportleitungen mit hohem Gasdurchsatz, ist es vorteilhaft, zusätzlich zu den obigen drei Parametern den Druck, die Temperatur und den Stickstoffanteil zu erfassen. Wenn eine geringere Meßgenauigkeit zulässig ist können für diesen Parameter auch geschätzte Werte benutzt werden.

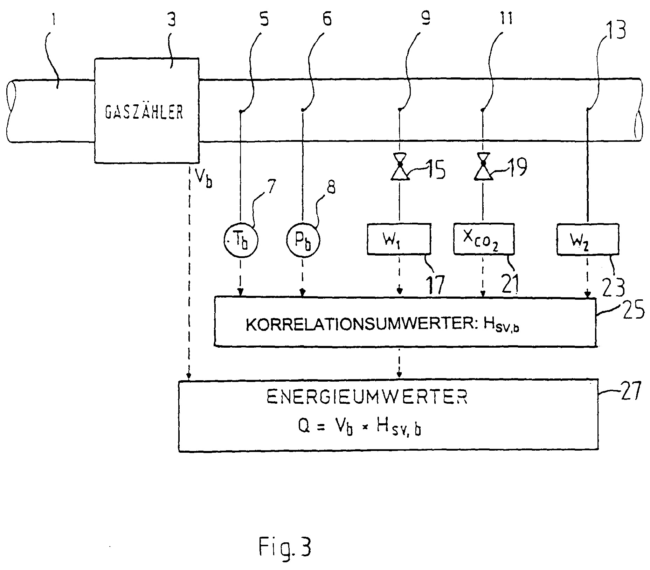

Fig. 3 zeigt eine Brenngasleitung 1, in der ein Gaszähler 3 angeordnet ist. In der Brenngasleitung 1 sind außerdem zwei Meßstellen 5 und 6 angeordnet. Der Meßstelle 5 ist ein Temperatursensor 7 und der Meßstelle 6 ein Drucksensor 8 zugeordnet. Ferner sind in der Brenngasleitung drei Entnahmestellen 9, 11 und 13 vorgesehen. Die Entnahmestelle 9 ist über ein Druckreduzierventil 15 mit einer Meßeinrichtung zur Messung der Schallgeschwindigkeit unter ersten Referenzbedingungen 17 verbunden. Die Entnahmestelle 11 ist über ein Druckreduzierventil 19 mit einer Meßeinrichtung zur Messung des Kohlenstoffdioxidanteils 21 verbunden. Entnahmestelle 13 ist mit einer Meßeinrichtung zur Messung der Schallgeschwindigkeit unter Betriebsbedingungen 23 verbunden.

Im Betriebszustand mißt der Gaszähler 3 den Volumenstrom und die Zeit und berechnet daraus das Volumen des zugeführten Brenngases. Der Temperatursensor 7 mißt die Temperatur und der Drucksensor 8 den Druck in der Brenngasleitung 1.

Die Signalausgänge des Temperatursensors 7, des Drucksensors 8 sowie der Meßeinrichtungen 17, 21 und 23 sind mit den Eingängen eines Korrelationsumwerter 25 verbunden zur korrelativen Bestimmung der Brennwert $H_{SV,b}$, entweder unmittelbar aus den erfaßten Meßgrößen oder über das oben beschriebene iterative Verfahren.

Der Ausgang dieses Korrelationsumwerters und der Signalausgang des Gaszählers 3 sind mit den Eingängen des Energieumwerters 27 verbunden. Anschließend berechnet der Energieumwerter 27 durch Multiplikation des Brennwertes $H_{SV,b}$ mit dem Volumen bei Betriebsbedingungen $V_b$ die Wärmemenge Q. In der praktischen Ausführung werden sind die Korrelationsumwertung und die Energieumwertung als hintereinander geschaltete Programmstufen in einem Rechner integriert.

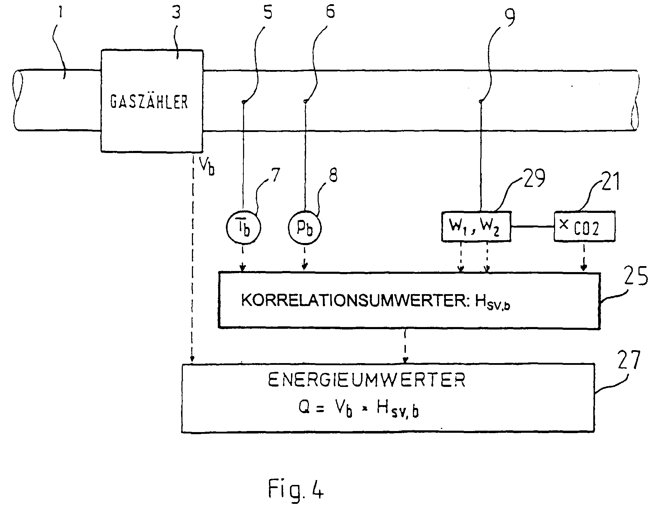

Fig. 4 zeigt eine Anordnung zur Durchführung eines zweiten Ausführungsbeispiels der Erfindung. Die Anordnung gemäß Fig. 4 unterscheidet sich dadurch von der Anordnung gemäß Fig. 3, daß beide Schallgeschwindigkeiten in eine Meßeinrichtung 29 zur Messung der Schallgeschwindigkeit erfasst werden. Das Brenngas wird ohne Druckreduzierung unmittelbar Meßeinrichtung 29 zugeführt, so daß die Schallgeschwindigkeit unter Betriebsbedingungen unmittelbar erfaßt werden kann. Meßeinrichtung 29 sind Mittel zugeordnet zum erhöhen oder erniedrigen des Druckes bis im Bereich der ersten oder zweiten Referenzbedingungen. Die Meßeinrichtung 29 ist über eine Gasleitung mit einer Meßeinrichtung 21 zur Bestimmung des Kohlenstoffdioxidanteils verbunden.

Die Wärmemenge wird über den Korrelationsumwerter 25 von dem Energieumwerter 27 bei diesem Ausführungsbeispiel, wie bei dem ersten Ausführungsbeispiel, auf der Basis des Volumens, der Temperatur und des Druckes bei Betriebsbedingungen, der Schallgeschwindigkeit unter ersten Referenzbedingungen, der Schallgeschwindigkeit unter Betriebsbedingungen sowie des Kohlenstoffdioxidanteils berechnet. Da in den beiden Ausführungsbeispielen gemäß Fig. 3 und Fig. 4 die Meßeinrichtungen für die Schallgeschwindigkeit und das Kohlenstoffdioxidanteil außerhalb der Brenngasleitung angeordnet sind, ist die Wartung dieser Einrichtungen sowie eine eventuelle Reparatur ohne besonderen technischen Aufwand durchzuführen. Außerdem sind nur verhältnismäßig wenige Referenzzyklen erforderlich, da die Schallgeschwindigkeiten unter vorgegebenen Bedingungen gemessen werden. Es besteht somit eine direkte Korrelation zwischen einer Änderung der Schallgeschwindigkeiten und einer Änderung der Gaszusammensetzung.

Das Ausführungsbeispiel gemäß Fig. 4 ist besonders kostengünstig zu realisieren, da die Schallgeschwindigkeit einfacher und günstiger zu messen ist als die Dichte des Brenngases, wie in den meisten bekannten Verfahren stattfindet.

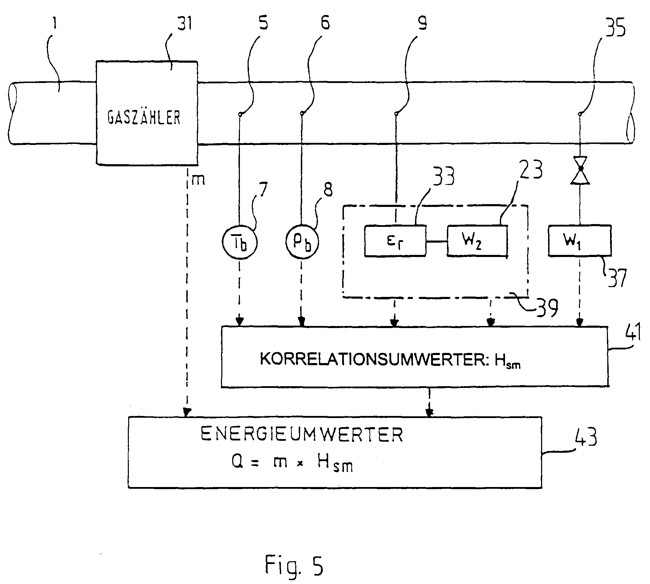

In Fig. 5 ist ein drittes Ausführungsbeispiel der Erfindung schematisch dargestellt. Die Anordnung unterscheidet sich von der Anordnung gemäß Fig. 4 dadurch, daß statt des Volumenzählers ein Massenzähler 31 zur Messung der Masse des zugeführten Brenngases vorgesehen ist und dass statt einer Meßeinrichtung zur Bestimmung des Kohlenstoffdioxidanteils eine Meßeinrichtung zur Bestimmung der Dielektrizitätskonstante 33 vorgesehen ist. Außerdem sind die Meßeinrichtungen zur Dielektrizitätsmessung 33 und zur Messung der Schallgeschwindigkeit unter Betriebsbedingungen 23 in einer gemeinsamen Meßumgebung 39 angeordnet. Ferner ist eine separate Entnamestelle 35 der Meßeinrichtung zur Bestimmung der Schallgeschwindigkeit unter ersten Referenzbedingungen 37 zugeordnet. Der Korrelationsumwerter 41 berechnet zunächst korrelativ den Brennwert $H_{Sm}$. Anschließend berechnet der Energieumwerter 43 durch Multiplikation des Brennwertes $H_{Sm}$ mit der Masse m die Warmemenge Q.

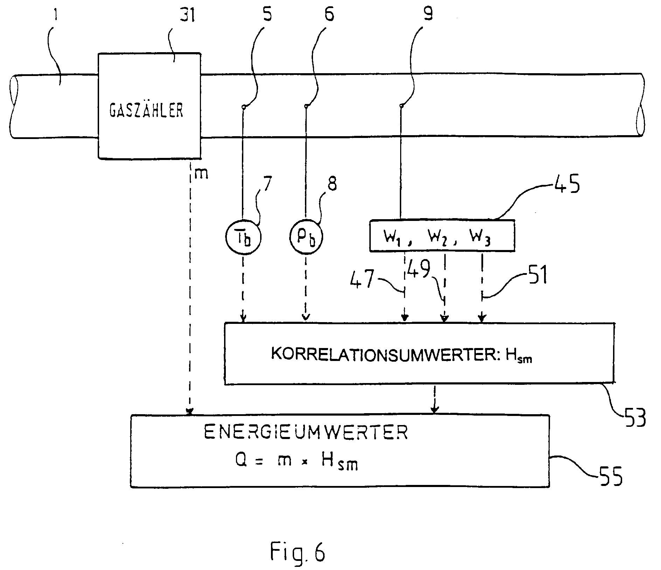

In dem in Fig. 6 dargestellte vierten Ausführungsbeispiel der Erfindung ist nur eine Meßeinrichtung 45 zur Messung der Schallgeschwindigkeit vorgesehen. In dieser Meßeinrichtung wird zuerst die Schallgeschwindigkeit unter Betriebsbedingungen 47 erfaßt und anschließend durch Einstellung der gewünschten Druckwerte die Schallgeschwindigkeit unter ersten und dritten Referenzbedingungen, 49 bzw. 51 erfaßt. Das weitere Verfahren läuft ab wie in den vorgehenden Ausführungsbeispielen über Korrelationsumwerter 53 und Energieumwerter 55.

Alle Ausführungsbeispiele haben den Vorteil, daß es nicht notwendig ist bezüglich der Schallgeschwindigkeit Messungen durchzuführen um die Wärmemenge zuverlässig zu bestimmen, weil die benötigten Daten abzuleiten sind aus den bekannten Beziehungen zwischen der Gaszusammensetzung und der Schallgeschwindigkeit. Diese bereits bekannten Daten können benutzt werden um den Brennwert aus der Schallgeschwindigkeit abzuleiten. Ferner hat die gemeinsame Meßumgebung für die Dielektrizitätsmessung bzw. die Messung des Kohlenstoffdioxidanteils und die Schallgeschwindigkeitsmessungen den Vorteil, daß nur eine Druck- und Temperaturmessung zusätzlich zur Druck- und Temperaturmessung in der Brenngasleitung benötigt wird. Das dritte Ausführungsbeispiel weist eine besonders hohe Genauigkeit bei der Bestimmung der Wärmemenge auf und ist daher besonders vorteilhaft. Es ist auch möglich einige der Einrichtungen zur Bestimmung der Meßgrößen unter Betriebsbedingungen innerhalb der Brenngasleitung anzuordnen.

**Patentansprüche**

1.    Verfahren zur verbrennungslosen Messung des Brennwertes von Brenngas, wobei

    a) die Schallgeschwindigkeit des Gases unter ersten und unter zweiten Referenzbedingungen bestimmt wird und eine der Meßgrößen Dielektrizitätskonstante, Schallgeschwindigkeit unter dritten Referenzbedingungen oder Kohlenstoffdioxidanteil des Brenngases erfaßt werden und
    b) aus diesen drei Parametern der Brennwert abgeleitet wird.

2.    Verfahren nach Anspruch 1, dadurch gekennzeichnet, daß die Dielektrizitätskonstante oder der Kohlenstoffdioxidanteil und wenigstens ein Wert für die Schallgeschwindigkeit in einer gemeinsamen Meßumgebung erfaßt werden.

3.    Verfahren nach Anspruch 1 oder 2, dadurch gekennzeichnet, daß als erste Referenzbedingungen für die Messung der Schallgeschwindigkeit ein Druck zwischen 1 und 10 bar und eine Temperatur oberhalb 225 K eingestellt wird.

4. Verfahren nach einem der Ansprüche 1 bis 3, dadurch gekennzeichnet, daß als zweite Referenzbedingungen für die Messung der Schallgeschwindigkeit ein Druck über 30 bar und eine Temperatur zwischen 225 K und 350 K eingestellt wird.

5. Verfahren nach einem der Ansprüche 4, dadurch gekennzeichnet, daß als dritte Referenzbedingungen für die Messung der Schallgeschwindigkeit ein Druck zwischen 175 und 225 bar und eine Temperatur zwischen 225 K und 350 K eingestellt wird, wobei der Druck bei der Messung unter zweiten Referenzbedingungen unter 70 bar eingestellt wird.

6. Verfahren nach einem der Ansprüche 1 bis 5, dadurch gekenzeichnet, daß als zweite Referenzbedingungen Betriebsbedingungen eingestellt werden.

7. Verfahren nach einem der Ansprüche 1 bis 6, dadurch gekennzeichnet, daß im Schritt a) zusätzlich wenigstens eine der Meßgrößen Temperatur, Druck oder der Stickstoffanteil erfaßt wird.

8. Verfahren nach Anspruch 1-7, dadurch gekennzeichnet, daß ein Teilstrom des Brenngases zur Messung einer der Schallgeschwindigkeiten abgezweigt wird und dass an diesem Teilstrom die Erfassung des Kohlenstoffdioxidanteils durchgeführt wird.

9. Verfahren nach einem der Ansprüche 1 bis 8, dadurch gekennzeichnet, daß den Verfahrensschritten a) und b) mehrere Meßzyklen vorgeschaltet werden, bei denen der Schritt a) mit mehreren Referenzgasen bekannten Brennwerts durchgeführt wird; daß aus dem verhältnis der bei den Referenzgasen erfaßten verschiedenen Meßsignale eine der Zahl der Meßzyklen entsprechende Zahl von Referenzsignalmustern in Zuordnung zu den bekannten Brennwerten gespeichert wird; und daß das Signalmuster aus einem späteren Meßzyklus an Brenngas unbekannten Brennwertes mit den Referenzsignalmustern zur Zuordnung eines bestimmten Brennwertes verglichen wird.

10. Verwendung des Verfahrens nach einem der Ansprüche 1 bis 9 zur verbrennungslosen Messung und/oder Regelung der Wärmemengenzufuhr zu Gasverbrauchseinrichtungen, insbesondere zu Erdgasverbrauchseinrichtungen, wobei im Schritt a) zusätzlich das Brenngas bzw. ein Teilstrom des Brenngases durch einen Volumen- oder Massenzähler geleitet und das Volumen bzw. die Masse des zugeführten Brenngases gemessen wird.

Fig. 1

(1) $w_1, p_1, T_1 \quad w_2, p_2, T_2 \quad w_3, p_3, T_3 \quad p_b, T_b$

(2) $H_{CH}\left(= H_{CH_4}\right), X_{CO_2}(= 0), X_{N_2}(= 0)$

B

(3) $X_{CH} = 1 - X_{N_2} - X_{CO_2}$

(4)
$$X_{CH_4} = X_{CH} - \left(X_{C_2H_6} + \cdots + X_{n-C_8H_{18}}\right)$$
$$X_{C_2H_6} = \left[\alpha_1 \cdot \left(H_{CH} - H_{CH_4}\right) + \beta_1 \cdot \left(H_{CH} - H_{CH_4}\right)^2\right] \cdot X_{CH}$$
$$\vdots$$
$$X_{n-C_8H_{18}} = \left[\alpha_9 \cdot \left(H_{CH} - H_{CH_4}\right) + \beta_9 \cdot \left(H_{CH} - H_{CH_4}\right)^2\right] \cdot X_{CH}$$

(5)
$$w_{1,ber} = f\left(p_1, T_1, X_{CH_4}, \cdots, X_{n-C_8H_{18}}, X_{CO_2}, X_{N_2}\right)$$
$$w_{2,ber} = f\left(p_2, T_2, X_{CH_4}, \cdots, X_{n-C_8H_{18}}, X_{CO_2}, X_{N_2}\right)$$
$$w_{3,ber} = f\left(p_3, T_3, X_{CH_4}, \cdots, X_{n-C_8H_{18}}, X_{CO_2}, X_{N_2}\right)$$

(6)
$$\Delta w_1 = \left|w_{1,ber} - w_1\right| \le 10^{-6}$$
$$\Delta w_2 = \left|w_{2,ber} - w_2\right| \le 10^{-6}$$
$$\Delta w_3 = \left|w_{3,ber} - w_3\right| \le 10^{-6}$$

Nein → A

Ja

(14)
ISO 6976 $\quad H_{S,n} = f_1\left(X_{CH_4}, \cdots, X_{n-C_8H_{18}}, X_{CO_2}, X_{N_2}\right)$
ISO 6976 $\quad \rho_n = f_2\left(X_{CH_4}, \cdots, X_{n-C_8H_{18}}, X_{CO_2}, X_{N_2}\right)$
ISO 12213 $\quad \rho_b = f_3\left(p_b, T_b, X_{CH_4}, \cdots, X_{n-C_8H_{18}}, X_{CO_2}, X_{N_2}\right)$
$\qquad H_{S,b} = H_{S,n} \cdot \rho_b / \rho_n$

(15) $H_{S,b}, H_{S,n}, \rho_b, \rho_n, X_{CH_4}, \cdots, X_{n-C_8H_{18}}, X_{N_2}, X_{CO_2}$

$$w^{-2} = \rho\beta_T - \frac{T\alpha^2}{C_p}$$

$$\alpha = V^{-1}\left(\frac{\partial V}{\partial p}\right)_T \quad \beta_T = -V^{-1}\left(\frac{\partial V}{\partial T}\right)_p \quad C_p = C_{p,0} - \int_0^p \rho^{-1}V^{-1}T\left(\frac{\partial^2 V}{\partial T^2}\right)_p dp$$

Fig. 2A

(B)

$$X_{CO_2} := X_{CO_2} + dX_{CO_2} \quad X_{N_2} := X_{N_2} + dX_{N_2} \quad H_{CH} := H_{CH} + dH_{CH}$$  (13)

$$\left(\frac{\partial w_1}{\partial X_{N_2}}\right) \cdot dX_{N_2} + \left(\frac{\partial w_1}{\partial X_{CO_2}}\right) \cdot dX_{CO_2} + \left(\frac{\partial w_1}{\partial H_{CH}}\right) \cdot dH_{CH} \approx w_1 - w_{1,ber}$$

$$\left(\frac{\partial w_2}{\partial X_{N_2}}\right) \cdot dX_{N_2} + \left(\frac{\partial w_2}{\partial X_{CO_2}}\right) \cdot dX_{CO_2} + \left(\frac{\partial w_2}{\partial H_{CH}}\right) \cdot dH_{CH} \approx w_2 - w_{2,ber}$$

$$\left(\frac{\partial w_3}{\partial X_{N_2}}\right) \cdot dX_{N_2} + \left(\frac{\partial w_3}{\partial X_{CO_2}}\right) \cdot dX_{CO_2} + \left(\frac{\partial w_3}{\partial H_{CH}}\right) \cdot dH_{CH} \approx w_3 - w_{3,ber}$$  (12)

$$\left(\frac{\partial w_1}{\partial X_{CO_2}}\right) = \frac{\left(w_{1,\Delta X_{CO_2}} - w_{1,ber}\right)}{\Delta X_{CO_2}} \quad \cdots \quad \left(\frac{\partial w_1}{\partial H_{CH}}\right) = \frac{\left(w_{1,\Delta H_{CH}} - w_{1,ber}\right)}{\Delta H_{CH}}$$

$$\left(\frac{\partial w_2}{\partial X_{CO_2}}\right) = \frac{\left(w_{2,\Delta X_{CO_2}} - w_{2,ber}\right)}{\Delta X_{CO_2}} \quad \cdots \quad \left(\frac{\partial w_2}{\partial H_{CH}}\right) = \frac{\left(w_{2,\Delta H_{CH}} - w_{2,ber}\right)}{\Delta H_{CH}}$$

$$\left(\frac{\partial w_3}{\partial X_{CO_2}}\right) = \frac{\left(w_{3,\Delta X_{CO_2}} - w_{3,ber}\right)}{\Delta X_{CO_2}} \quad \cdots \quad \left(\frac{\partial w_3}{\partial H_{CH}}\right) = \frac{\left(w_{3,\Delta H_{CH}} - w_{3,ber}\right)}{\Delta H_{CH}}$$  (11)

(A)

$$w_{1,\Delta X_{CO_2}} = f\left(p_1, T_1, X_{CH_4}, \cdots, X_{n-C_8H_{18}}, X_{CO_2}', X_{N_2}\right)$$

$$w_{1,\Delta X_{N_2}} = f\left(p_1, T_1, X_{CH_4}, \cdots, X_{n-C_8H_{18}}, X_{CO_2}, X_{N_2}'\right)$$

$$w_{1,\Delta H_{CH}} = f\left(p_1, T_1, X_{CH_4}', \cdots, X_{n-C_8H_{18}}', X_{CO_2}, X_{N_2}\right)$$

$$\vdots$$

$$w_{3,\Delta H_{CH}} = f\left(p_3, T_3, X_{CH_4}', \cdots, X_{n-C_8H_{18}}', X_{CO_2}, X_{N_2}\right)$$  (10)

$$X_{CH_4}' = X_{CH} - \left(X_{C_2H_6}' + \cdots + X_{n-C_8H_{18}}'\right)$$

$$X_{C_2H_6}' = \left[\alpha_1 \cdot \left(H_{CH}' - H_{CH_4}\right) + \beta_1 \cdot \left(H_{CH}' - H_{CH_4}\right)^2\right] \cdot X_{CH}'$$

$$\vdots$$

$$X_{n-C_8H_{18}}' = \left[\alpha_9 \cdot \left(H_{CH}' - H_{CH_4}\right) + \beta_9 \cdot \left(H_{CH}' - H_{CH_4}\right)^2\right] \cdot X_{CH}'$$  (9)

$$X_{CH}' = 1 - X_{N_2}' - X_{CO_2}'$$  (8)

$$X_{CO_2}' = X_{CO_2} + \Delta X_{CO_2} \quad X_{N_2}' = X_{N_2} + \Delta X_{N_2} \quad H_{CH}' = H_{CH} + \Delta H_{CH}$$  (7)

Fig. 2B

Fig. 3

Fig. 4

Fig. 5

Fig. 6